Europäisches Patentamt

European Patent Office

Office européen des brevets

(19)

(11) Veröffentlichungsnummer : **0 135 007**
**B1**

(12) # EUROPÄISCHE PATENTSCHRIFT

(45) Veröffentlichungstag der Patentschrift :
10.09.86

(21) Anmeldenummer : 84108172.2

(22) Anmeldetag : 12.07.84

(51) Int. Cl.⁴ : **C 07 C139/14, C 07 C143/02**

(54) **Verfahren zur schonenden Isolierung von Paraffinsulfonat und Schwefelsäure aus Paraffin-Sulfoxidations-Reaktionsgemischen.**

(30) Priorität : 15.07.83 DE 3325516

(43) Veröffentlichungstag der Anmeldung :
27.03.85 Patentblatt 85/13

(45) Bekanntmachung des Hinweises auf die Patenterteilung : 10.09.86 Patentblatt 86/37

(84) Benannte Vertragsstaaten :
DE FR GB IT NL

(56) Entgegenhaltungen :
DE-A- 1 568 591
DE-A- 2 139 477
DE-A- 2 745 691
DE-A- 2 855 849
DE-A- 3 004 651
DE-A- 3 013 808
US-A- 4 321 216

(73) Patentinhaber : HOECHST AKTIENGESELLSCHAFT
Postfach 80 03 20
D-6230 Frankfurt am Main 80 (DE)

(72) Erfinder : Pistorius, Rudolf, Dr.
Neumühlstrasse 15
D-6274 Hünstetten (DE)

EP 0 135 007 B1

## Beschreibung

Die durch Sulfoxidation von n-Paraffinen z. B. nach dem Verfahren des deutschen Patents 910 165 erhältlichen wässrigen Lösungen von Paraffinsulfonsäuren enthalten außerdem noch Schwefeldioxid, Schwefelsäure und hydrotrop gelöste Paraffine. Um aus solchen Reaktionsgemischen brauchbare Paraffinsulfonsäuren bzw. Paraffinsulfonate von guter Qualität, d.h. möglichst schwefelsäure- bzw. salzarme, helle und weitestgehend geruchsfreie Produkte zu isolieren, müssen Schwefeldioxid, Schwefelsäure und Paraffine möglichst quantitativ und schonend abgetrennt werden. Die Paraffinsulfoxidationsprodukte beginnen sich bereits bei Temperaturen oberhalb 50 °C zu zersetzen, was sich äußerlich an der Verfärbung des sauren Reaktionsgemisches von wasserhell über gelblich, braun bis schließlich tiefschwarz zeigt. Wenn auch die Menge der durch die Temperatureinwirkung zersetzten Paraffinsulfonsäure noch relativ gering ist, solange die sauren Reaktionsgemische nicht über längere Zeit Temperaturen über 100 °C ausgesetzt sind, erfordert jedoch bereits ein kleiner Anteil an zersetzten Produkten wegen ihrer Farbintensität einen beträchtlichen Bleichaufwand, wenn man zu einwandfrei hellen Produkten kommen will.

Es wurde festgestellt, daß demgegenüber die schwach alkalisch reagierenden Salze der Paraffinsulfonsäuren relativ stabil sind. Temperaturen unter 200 °C führen auch bei längerer Erhitzungsdauer nur zu ganz unwesentlichen Verfärbungen und auch höhere Temperaturen bis zu etwa 260 °C ergeben Verfärbungen, die sich noch leicht mit geringen Bleichmittelmengen wieder beseitigen lassen.

Es muß daher bereits beim ersten Schritt der Aufarbeitung der Paraffin-Sulfoxidations-Reaktionsgemische, bei der Entgasung zur Entfernung des Schwefeldioxids, darauf geachtet werden, daß keine Verfärbung auftritt. Wird die Entgasung im schwachen Vakuum ausgeführt, so bedarf es nur eines kurzzeitigen Erwärmens auf etwa 85 °C, um eine nahezu vollständige Eliminierung des Schwefeldioxids zu erreichen. Durch unmittelbar anschließendes Wiederabkühlen des Reaktionsgemisches auf Raumtemperatur kann bei diesem Prozeß eine merkliche Zersetzung, d.h. eine eintretende Farbvertiefung des Reaktionsgemisches verhindert werden.

Im Hinblick auf die Qualität des Paraffinsulfonats wäre es günstif, das Reaktionsgemisch nach der Entgasung sofort zu neutralisieren. Eine derartige Verfahrensweise ist jedoch wegen des zur Neutralisierung der Schwefelsäure erforderlichen hohen Verbrauchs an Alkali sowie wegen der beachtlichen Verluste an Paraffinsulfonat, die beim Abfiltrieren des Alkalisulfats auftreten, unwirtschaftlich und technisch nicht praktikabel.

Nach der Entfernung des Schwefeldioxids aus dem Reaktionsgemisch muß deshalb versucht werden, vor der Neutralisation die Schwefelsäure möglichst vollständig und unter Schonung der Paraffinsulfonsäure aus dem Gemisch abzutrennen. Bei den bekannten Verfahren, die ein solches Ziel anstreben, geht man im allgemeinen so vor, daß man das entgaste Sulfoxidationsgemisch mit einem geeigneten organischen Lösungsmittel behandelt, um eine Entmischung in eine organische Phase, welche die Paraffinsulfonsäuren enthält, und in eine wäßrige Phase, welche die Schwefelsäure soweit wie möglich in Form einer im allgemeinen 10 bis 25 %igen wäßrigen Lösung enthält, herbeizuführen. Die beiden Phasen werden dann getrennt und die organische Phase zur Isolierung der Paraffinsulfonsäuren bzw. ihrer Salze weiter aufgearbeitet. So ist es aus der am 29.1.1953 bekanntgemachten deutschen Patentanmeldung F 3718, 120 bereits bekannt, wasserunlösliche oder nur beschränkt mit Wasser mischbare organische Lösungsmittel, wie z. B. Benzol, Chlorbenzol, Cyclohexan, Tetrachlorkohlenstoff, Chloroform, Methylenchlorid und dergleichen zur Abtrennung von Schwefelsäure dem Sulfoxidationsgemisch zuzusetzen. Gemäß DE-OS 27 30 245 kommen für den gleichen Zweck auch Ether wie z. B. Diethylether oder Di-n-butylether und gemäß DE-OS 27 45 691 auch Ketone oder Ester sowie gemäß DE-OS 21 39 477 auch Alkohole mit mindestens 5 Kohlenstoffatome zur Anwendung.

Keines dieser bekannten Verfahren zur Abtrennung von Schwefelsäure bei niederen Temperaturen hat sich bislang technisch durchsetzen können, weil entweder der Anteil der abgeschiedenen Schwefelsäure zu gering oder aber der Aufwand zur destillativen Abtrennung des Lösungsmittels zu groß ist.

Überraschend wurde nun gefunden, daß die wäßrige Schwefelsäure aus dem entgasten Paraffin-Sulfoxidations-Reaktionsgemisch besonders weitgehend bei niederer Temperatur abgeschieden wird, wenn man das Reaktionsgemisch mit einem polaren Alkohol mit 2 bis 8 C-Atomen und einem organischen, wenig polaren, wasserunlöslichen oder beschränkt mit Wasser mischbaren Lösungsmittel mischt.

Daneben ist auch aus der DE-A1-3 013 808 bekannt, die Schwefelsäure nicht allein mit Hilfe eines Alkohols mit mehr als 5 C-Atomen abzutrennen, sondern mit einer Mischung aus einem solchen Alkohol und Paraffin. Dieses Verfahren ist aber nachteilig, weil dadurch ein weiterer Hilfsstoff benötigt wird, der im weiteren Verlauf der Aufarbeitung wieder entfernt werden muß. In der US-A-4 321 216 ist die Aufarbeitung von Reaktionsgemischen bei der Herstellung von Petrolsulfonaten beschrieben, wobei Lösemittelsysteme aus einer nicht-mischbaren polaren und einer nicht-polaren Komponente benutzt werden.

Gegenstand der Erfindung ist somit ein Verfahren zur schonenden Isolierung von Paraffinsulfonat aus den bei der Sulfoxidation von n-Paraffinen anfallenden, durch Entgasung vom Schwefeldioxid befreiten Reaktionsgemischen, die höhermolekulare Paraffinsulfonsäuren, Schwefelsäure, n-Paraffin und Wasser enthalten, welches dadurch gekennzeichnet ist, daß man auf solche Reaktionsgemische aliphatische Alkohole mit 2 bis 8 Kohlenstoffatomen und wasserunlösliche oder nur beschränkt mit Wasser mischbare organische Lösungsmittel aus der Gruppe Tetrachlorkohlenstoff, Chloroform, 1,2-Dichloräthan, 1,1,2-Trichlor-trifluoräthan und Methylenchlorid zugleich einwirken läßt, die abgeschiedene, wäßrige Schwefelsäure enthaltende Phase abtrennt und die Paraffinsulfonate enthaltende Produktphase neutralisiert, eindampft und mit überhitztem Wasserdampf das restliche Paraffin austreibt.

Als Ausgangsmaterial für das Verfahren der vorliegenden Erfindung können die durch schonende Entgasung von Schwefeldioxid befreiten Reaktionsgemische der bekannten Sulfoxidationsverfahren eingesetzt werden, die wäßrige Gemische von höhermolekularen Paraffinsulfonsäuren, Schwefelsäure und n-Paraffin darstellen. Beispielsweise können die nach dem Verfahren der deutschen Patentschrift 735 096 erhältlichen Reaktionsgemische verwendet werden. Es kann ferner auch von Reaktionsgemischen ausgegangen werden, wie sie bei der Sulfoxidation mit Hilfe von Peroxiden, Ozon oder auch mit $\gamma$-Strahlen, z. B. nach dem Verfahren der deutschen Patentschrift 11 39 116 erhalten werden. Bevorzugt legt man für das vorliegende Verfahren die bei der Sulfoxidation von n-Paraffinen mit Kettenlängen von etwa 7 bis 20, vorzugsweise 13 bis 18 Kohlenstoffatomen anfallenden wäßrigen Reaktionsgemische zugrunde, die sich bei der Sulfoxidation als klare schwerere Phase von dem überschüssigen Paraffin abtrennen.

Gemäß der Erfindung können die aliphatischen Alkohole und die weniger polaren organischen Lösungsmittel den von Schwefeldioxid befreiten Reaktionsgemischen gleichzeitig oder in beliebiger Reihenfolge nacheinander zugemischt werden. Die zuzusetzende Menge an aliphatischen Alkoholen beträgt, in Abhängigkeit von der Zusammensetzung des Reaktionsgemisches, etwa 5 bis 80 Gew.-%, vorzugsweise 8 bis 50 Gew.-%, insbesondere 10 bis 45 Gew.-%, bezogen auf das Gewicht des eingesetzten Reaktionsgemisches. Die zuzusetzende Menge des schwach polaren organischen Lösungsmittels liegt im Bereich von etwa 10 bis 200 Gew.-%, vorteilhaft von 20 bis 150 Gew.-% insbesondere bei 30 bis 70 Gew.-%, bezogen auf das Gewicht der eingesetzten Reaktionsmischung. Dabei sollte jedoch die Menge des schwach polaren organischen Lösungsmittels vorzugsweise gleich groß oder größer, insbesondere etwa doppelt so groß sein wie die Menge des verwendeten Alkohols.

Die Einwirkung von Alkohol und organischem Lösungsmittel auf das Reaktionsgemisch erfolgt bei niederen Temperaturen, zweckmäßig im Bereich von etwa 5 bis 50 °C, vorzugsweise bei etwa 10 bis 30 °C. Die Ausbildung der beiden Phasen erfolgt innerhalb von längstens 2 Stunden, im allgemeinen jedoch bereits nach 15 bis 30 Minuten. Die Phasentrennungszeit kann durch geeignete Maßnahmen z. B. durch Passieren des Gemisches durch einen Tröpfchenabscheider entscheidend verkürzt werden. Bereits die Filtration durch ein Papierfilter oder durch Glaswolle bewirkt eine wesentlich schnellere Trennung, so daß bereits nach etwa 10 Minuten die wäßrige, Schwefelsäure enthaltende Phase abgeschieden werden kann.

Bei dem Verfahren der Erfindung können als weniger polare, wasserunlösliche oder beschränkt mit Wasser mischbare Lösungsmittel solche Verwendung finden, die unter den Bedingungen der Aufarbeitung gegenüber den Bestandteilen des Reaktionsgemisches inert sind. Es kommen organische Lösungsmittel in Betracht, deren Siedepunkt im Bereich von etwa 20 bis 150 °C, vorzugsweise von 30 bis 100 °C liegt. Die geeigneten Lösungsmittel dieser Art sind Tetrachlorkohlenstoff, Chloroform, 1,2-Dichloräthan, 1,1,2-Trichlor-trifluoräthan und insbesondere Methylenchlorid. Geeignete aliphatische Alkohole sind z. B. Ethanol, Propanole, Butanole, Hexanole und 2-Ethylhexanol. Bevorzugt zur Anwendung kommen n-Hexanol und 2-Ethylhexanol.

Nach dem Abtrennen der wäßrigen Schwefelsäure wird die die Paraffinsulfonsäuren, Alkohol, organisches Lösungsmittel und noch Paraffin enthaltende Produktphase bei Temperaturen unter 55 °C, vorzugsweise unter 50 °C vorteilhaft so neutralisiert, daß unter intensivem Rühren gleichzeitig Alkali und die Produktphase zusammengegeben werden. Dabei wird die Dosiergeschwindigkeit so eingestellt, daß der pH-Wert, z. B. gemessen mit einer geeichten pH-Glaselektrode, im Gemisch der beiden Flüssigkeiten immer oberhalb 7, insbesondere oberhalb 10,5 liegt, damit das schließlich am Ende der Aufarbeitung erhaltene Paraffinsulfonat einen neutralen bis schwach alkalischen pH-Wert aufweist. Es ist aber auch möglich, bei der Neutralisation die gesamte Alkalimenge vorzulegen und die Produktphase solange zulaufen zu lassen, bis der gewünschte pH-Wert oberhalb pH 7 bzw. oberhalb pH 10,5 erreicht ist. Grundsätzlich möglich, wenn auch weniger vorteilhaft ist ein umgekehrtes Vorgehen, d. h. Zugabe des Alkalis zur vorgelegten Produktlösung.

Zur Neutralisation der Paraffinsulfonsäuren können beliebige Alkalien verwendet werden. Es können Natrium- oder Kaliumhydroxid, zweckmäßig in Form konzentrierter wässriger Lösungen genommen werden ; es ist jedoch auch möglich, Natrium- oder Kaliumcarbonat oder Natrium- oder Kaliumbicarbonat einzusetzen.

Nach der Neutralisation wird die Produktphase zur Entfernung von Alkohol, organischem Lösungsmittel und Paraffin in an sich bekannter Weise durch ein- oder mehrstufige Destillation eingedampft, wobei schließlich noch mit überhitztem Wasserdampf von 180 bis 280 °C das restliche Paraffin und gegebenenfalls Lösungsmittel ausgetrieben wird. Die so gewonnene Paraffinsulfonat-Schmelze kann anschließend durch Zugabe von Wasser bei 100 bis 160 °C unter entsprechendem Druck auf die gewünschte Konzentration eingestellt werden, wobei man dem Wasser zur Beseitigung eventueller Farb- und Geruchsspuren noch 0,01 bis 1 Gew.-%, insbesondere 0,1 bis 0,3 Gew.-% Wasserstoffperoxid zumischen kann.

# 0 135 007

Ein wesentlicher Vorteil des erfindungsgemäßen Verfahrens ist darin zu sehen, daß es hiermit gelingt, helle, geruchs- und salzarme Produkte auf wirtschaftliche Weise zu erhalten, was vor allem auf die weitgehende Abscheidung der Schwefelsäure unter außerordentlich milden Bedingungen bewirkt wird. Die bei dem Verfahren der Erfindung erreichte weitgehende Abscheidung der Schwefelsäure durch Einwirkung von Alkoholen und weniger polaren organischen Lösungsmitteln ist deshalb überraschend, weil jedes dieser Lösungsmittel für sich allein nur eine weit unvollständigere oder auch überhaupt keine Abscheidung von Schwefelsäure bewirkt, so daß dieser Kombinationseffekt nicht zu erwarten war. Wegen der gegenüber Alkoholen wesentlich kleineren Verdampfungsenergie der schwach polaren organischen Lösungsmittel erweist es sich weiterhin als vorteilhaft, daß bereits Alkoholmengen unter 40 Gew.-%, in geeigneten Fällen auch unter 20 Gew.-% (bezogen auf die Menge des Reaktionsgemisches) ausreichen, um Paraffinsulfonate mit einem Gehalt an anorganischen Salzen unter 5 % (bezogen auf die waschaktive Substanz) zu erhalten.

## Beispiel 1

In einer Sulfoxidationsapparatur wurden geradkettige Paraffinkohlenwasserstoffe mit 14 bis 17 Kohlenstoffatomen mit Schwefeldioxid und Sauerstoff in Gegenwart von Wasser unter Bestrahlung mit UV-Licht bei 30 bis 40 °C umgesetzt. Es wurde ein wäßriges Reaktionsgemisch erhalten, das durch Erwärmen auf 85 °C unter schwachem Vakuum von Schwefeldioxid befreit wurde. Hiernach bestand das Reaktionsgemisch aus 42,3 % Wasser, 7,33 % Schwefelsäure, 21,25 % Paraffinsulfonsäuren und 29,12 % Paraffinen. Proben dieses Reaktionsgemisches wurden jeweils allein mit n-Hexanol, allein mit Methylenchlorid und mit n-Hexanol/Methylenchlorid-Gemischen gemäß nachfolgender Tabelle versetzt. Nach 12stündigem Absetzen bei 23 °C wurde die abgeschiedene wäßrige Schwefelsäure abgetrennt. Die erhaltenen Produktphasen wurden durch gleichzeitiges Zusammengeben mit Natronlauge auf pH 12,5 eingestellt und anschließend bei Temperaturen von 160 °C (± 20 °C) bis zur Schmelze eingedampft, die noch mit überhitztem Wasserdampf gestrippt wurde, bis kein Paraffin mehr überging. Anschließend wurde bei 120 °C soviel Wasser zugegeben, daß Paraffinsulfonatpasten mit 60 Gew.-% Paraffinsulfonat entstanden, in denen der Gehalt an Natriumsulfat bestimmt wurde.

In Abhängigkeit der Lösungsmittelmengen (bezogen auf das Reaktionsgemisch) wurden folgende Natriumsulfat-Gehalte gefunden:

### Tabelle 1

Gehält an $Na_2SO_4$ in Gew.-% (bezogen auf 60 % waschaktive Substanz)

| n-Hexanol-zusatz (Gew.-%) | Methylenchlorid-Zusatz (Gew.-%) | | | | | | |
|---|---|---|---|---|---|---|---|
| | 0 | 10 | 15 | 20 | 25 | 30 | 40 |
| 0 | 27,8 | n.b. | n.b. | n.b. | n.b. | 18,99 | 16,55 |
| 10 | 5,51 | 4,1 | 3,1 | 2,84 | n.b. | 2,37 | n.b. |
| 15 | 3,35 | 2,6 | 2,03 | 1,89 | 1,84 | 1,59 | n.b. |
| 20 | 2,73 | 2,04 | 1,56 | 1,47 | 1,39 | 1,26 | n.b. |
| 25 | 2,09 | 1,78 | 1,28 | 1,40 | 1,30 | 1,17 | n.b |
| 30 | 1,73 | 1,6 | 1,15 | 1,09 | 0,88 | 0,79 | n.b. |
| 35 | 2,00 | 0,96 | 1,07 | 0,96 | 0,86 | n.b. | n.b. |
| 40 | 2,01 | 0,91 | 1,21 | 1,12 | 1,22 | n.b. | n.b. |

n. b. = nicht bestimmt

## Beispiel 2

Proben eines von Schwefeldioxid befreiten Reaktionsgemisches der gleichen Zusammensetzung wie in Beispiel 1 angegeben, wurden mit Isobutanol und Methylenchlorid bzw. mit Isobutanol und 1,1,2-Trichlortrifluoräthan (F 113) gemäß nachfolgender Tabelle 2 versetzt. Nach der Aufarbeitung zu 60 %igen Paraffinsulfonatpasten wie in Beispiel 1 angegeben wurden in den erhaltenen Produkten folgende Gewichtsanteile an Natriumsulfat (bezogen auf 60 % Waschaktivsubstanz) gefunden:

Tabelle 2

Gehalt an $Na_2SO_4$ in Gew.-%

| Isobutanol-Zusatz (Gew.-%) | Methylenchlorid-Zusatz (Gew.-%) | | | F 113-Zusatz (Gew.-%) | |
|---|---|---|---|---|---|
| | 0 | 30 | 50 | 30 | 50 |
| 10 | 7,29 | n.b. | 2,22 | n.b. | 4,57 |
| 15 | 4,20 | 2,01 | n.b. | 3,33 | n.b. |

n. b. = nicht bestimmt

**Patentansprüche**

1. Verfahren zur schonenden Isolierung von Paraffinsulfonat aus wäßrigen, bei der Sulfoxidation von n-Paraffinen anfallenden Reaktionsgemischen, die durch Entgasung von Schwefeldioxid befreit wurden und die höhermolekulare Sulfonsäuren, Schwefelsäure, n-Paraffine und Wasser enthalten, dadurch gekennzeichnet, daß man auf die Reaktionsgemische aliphatische Alkohole mit 2 bis 8 Kohlenstoffatomen und wasserunlösliche oder nur beschränkt mit Wasser mischbare organische Lösungsmittel aus der Gruppe Tetrachlorkohlenstoff, Chloroform, 1,2-Dichloräthan, 1,1,2-Trichlor-trifluoräthan und Methylenchlorid zugleich einwirken läßt, die abgeschiedene, wäßrige Schwefelsäure enthaltende Phase abtrennt und die Paraffinsulfonate enthaltende Produktphase neutralisiert, eindampft und die überhitztem Wasserdampf das restliche Paraffin austreibt.

2. Verfahren nach Anspruch 1, dadurch gekennzeichnet, daß die Einwirkung der Alkohole und organischen Lösungsmittel zur Abscheidung der wäßrigen Schwefelsäure bei Temperaturen unter 50 °C erfolgt.

3. Verfahren nach Anspruch 1, dadurch gekennzeichnet, daß die Alkohole und organischen Lösungsmittel im Gewichtsverhältnis 1 : 1 bis 1 : 2 angewandt werden.

4. Verfahren nach Anspruch 1, dadurch gekennzeichnet, daß als wasserunlösliches oder nur beschränkt mit Wasser mischbares organisches Lösungsmittel Methylenchlorid zugesetzt wird.

5. Verfahren nach Anspruch 1, dadurch gekennzeichnet, daß als aliphatischer Alkohol n-Hexanol oder 2-Ethylhexanol zugegeben wird.

**Claims**

1. A process for the isolation under mild conditions of paraffinsulfonate from the aqueous reaction mixtures which result from the sulfoxidation of n-paraffins, from which sulfur dioxide has been removed by degassing, and which contain higher molecular weight sulfonic acids, sulfuric acid, n-paraffins and water, which process comprises allowing aliphatic alcohols having 2 to 8 carbon atoms and organic solvents which are insoluble in water or are miscible with water to only a limited extent of the group consisting of carbon tetrachloride, chloroform, 1,2-dichloroethane, 1,1,2-trichlorotrifluoroethane and methylene chloride to act on such reaction mixtures at the same time, removing the aqueous phase which has separated out and contains sulfuric acid, and neutralizing the product phase containing paraffinsulfonates, evaporating it and driving out the remaining paraffin using superheated steam.

2. The process as claimed in claim 1, wherein the action of the alcohols and organic solvents to separate out the sulfuric acid takes place at temperatures below 50 °C.

3. The process as claimed in claim 1, wherein the alcohols and organic solvents are used in the weight ratio 1 : 1 to 1 : 2.

4. The process as claimed in claim 1, wherein methylene chloride is added as the organic solvent which is insoluble in water or is miscible with water to only a limited extent.

5. The process as claimed in claim 1, wherein n-hexanol or 2-ethylhexanol is added as the aliphatic alcohol.

**Revendications**

1. Procédé d'isolement ménagé de paraffine-sulfonate à partir de mélanges réactionnels aqueux, se formant lors de la sulfoxydation de n-paraffines, qui ont été débarrassés de l'anhydride sulfureux par dégazage et qui contiennent des acides sulfoniques de masse moléculaire élevée, de l'acide sulfurique,

**0 135 007**

des n-paraffines et de l'eau, caractérisé en ce qu'on fait agir simultanément sur les mélanges réactionnels des alcools aliphatiques en $C_2$ à $C_8$ et des solvants organiques insolubles dans l'eau ou ne présentant qu'une miscibilité limitée avec l'eau du groupe du tétrachlorure de carbone, du chloroforme, du 1,2-dichloréthane, du 1,1,2-trichloro-trifluoréthane et du chlorure de méthylène, en ce qu'on sépare la phase contenant de l'acide sulfurique aqueux qui s'est séparée, en ce qu'on neutralise la phase produit contenant les paraffinesulfonates et en ce qu'on chasse la paraffine résiduelle avec de la vapeur d'eau surchauffée.

2. Procédé suivant la revendication 1, caractérisé en ce que l'action des alcools et des solvants organiques pour la séparation de l'acide sulfurique aqueux s'effectue à des températures inférieures à 50 °C.

3. Procédé suivant la revendication 1, caractérisé en ce que les alcools et les solvants organiques sont utilisés dans le rapport pondéral de 1 : 1 à 1 : 2.

4. Procédé suivant la revendication 1, caractérisé en ce qu'on ajoute comme solvant organique insoluble dans l'eau ou ne présentant qu'une miscibilité limitée avec l'eau, du chlorure de méthylène.

5. Procédé suivant la revendication 1, caractérisé en ce qu'on ajoute comme alcool aliphatique du n-hexanol ou du 2-éthylhexanol.

6